# EUROPÄISCHE PATENTSCHRIFT

(11) **EP 0 918 050 B1**
(45) Veröffentlichungstag und Bekanntmachung des Hinweises auf die Patenterteilung: **12.12.2001**
(21) Anmeldenummer: 98117331.3
(22) Anmeldetag: 12.09.1998
(51) Int. Cl.: C07C 217/90, C07D 213/69

(54) **Bis-o-amino(thio)phenole und deren Herstellung**
Bis-o-amino(thio)phenols and their preparation
Bis-o-amino(thio)phénoles et leur préparation

(30) Priorität: 24.09.1997 DE 19742196
(43) Veröffentlichungstag der Anmeldung: 26.05.1999
(73) Patentinhaber: Infineon Technologies AG, 81669 München (DE)
(72) Erfinder: Sezi, Recai Dr., 91341 Röttenbach (DE); Weber, Andreas Dr., 92289 Ursensollen (DE); Keitmann, Michael, 91074 Herzogenaurach (DE)
(74) Vertreter: Müller . Hoffmann & Partner Patentanwälte

(56) Entgegenhaltungen:
- EP-A- 0 023 662
- EP-A- 0 300 326
- DE-A- 19 537 893
- CHEMICAL ABSTRACTS, vol. 106, no. 10, 9. März 1987 Columbus, Ohio, US; abstract no. 67818, IGNATENKO, N. M. ET AL: "Bis(3-amino-4-hydroxyphenoxy)perfluoroary lenes as monomers for producing polybenzoxazoles with higher thermal and hydrolytic stability" XP002088350 & SU 1 205 518 A (INSTITUTE OF PHYSICAL-ORGANIC CHEMISTRY AND COAL CHEMISTRY, KIEV, USSR)
- CHEMICAL ABSTRACTS, vol. 120, no. 14, 4. April 1994 Columbus, Ohio, US; abstract no. 164950, WINZELER, JACQUELINE T. ET AL: "The synthesis of 6F bis(o-aminophenol) monomers by a nucleophilic substitution reaction" XP002088351 & POLYM. PREPR. (AM. CHEM. SOC., DIV. POLYM. CHEM.) (1993), 34(1), 425-6 CODEN: ACPPAY;ISSN: 0032-3934,1993,
- CHEMICAL ABSTRACTS, vol. 92, no. 4, 28. Januar 1980 Columbus, Ohio, US; abstract no. 23145, ADAM, CORNELIU ET AL: "Polybenzothiazolimide" XP002088424 & RO 63 916 A (ROM.)

## Beschreibung

Die Erfindung betrifft neue Bis-o-aminophenole und Bis-o-aminothiophenole, die zusammen kurz auch als Bis-o-amino-(thio)phenole bezeichnet werden, sowie ein Verfahren zu deren Herstellung.

Bis-o-aminophenole werden insbesondere zur Herstellung von hochtemperaturstabilen Polymeren, wie Polybenzoxazolen (PBO) bzw. deren Vorstufen, sowie zur Herstellung von Hydroxypolyimiden benötigt (siehe dazu beispielsweise EP 0 264 678 B1 und EP 0 300 326 B1). PBO-Vorstufen können in der Weise hergestellt werden, daß ein Dicarbonsäurechlorid mit einem Bis-o-aminophenol umgesetzt wird. Während aber wegen der Vielfalt industrieller Einsatzmöglichkeiten zahlreiche Dicarbonsäuren bzw. deren Chloride verfügbar sind, gibt es vergleichsweise wenige Bis-o-aminophenole. Außerdem beeinflußt die Art des eingesetzten Aminophenols in starkem Maße das Eigenschaftsspektrum des damit hergestellten Polymers. So werden nicht nur das thermische, elektrische und mechanische Verhalten, sondern auch die Löslichkeit und die Hydrolysestabilität sowie zahlreiche weitere Eigenschaften des Polymers durch das bei der Herstellung verwendete Aminophenol stark beeinflußt.

PBO-Vorstufen können in Form einer photosensitiven Zusammensetzung kostengünstig auf direktem Weg, d.h. ohne einen Hilfsresist, strukturiert werden. Im Vergleich mit anderen direkt photostrukturierbaren Dielektrika, wie Polyimid (PI) und Benzocyclobuten (BCB), bieten PBO-Vorstufen den Vorteil der positiven Strukturierbarkeit und der wäßrig-alkalischen Entwicklung (siehe EP 0 023 662 B1 und EP 0 264 678 B1). Hierzu müssen die verwendeten PBO-Vorstufen bei der Belichtungswellenlänge weitgehend transparent und im - vorzugsweise metallionenfreien - Entwickler ausreichend löslich sein. Wie die Polyimide haben auch Polybenzoxazole den großen Vorteil, daß sie - im Vergleich zum cyclisierten Endprodukt - als gut lösliche Vorstufe auf ein Substrat aufgebracht und anschließend cyclisiert werden können, wobei die Löslichkeit und damit die Sensibilität gegenüber Lösemitteln und anderen Prozeßchemikalien stark abnimmt.

Für den Einsatz von Polybenzoxazolen in der Mikroelektronik, insbesondere als Dielektrikum zwischen zwei Metallebenen, beispielweise bei Multi-Chip-Modulen sowie Speicher- und Logikchips, oder als Pufferschicht ("Buffercoat") zwischen dem Chip und seinem Gehäuse, ist neben guten elektrischen, mechanischen und thermischen Eigenschaften auch eine geringe Feuchteaufnahme erforderlich; der Feuchteanteil in der Polymerschicht beeinträchtigt nämlich einerseits die elektrischen Eigenschaften des Polymers und kann andererseits bei hohen Temperaturen zu Blasenbildungen und Abplatzungen führen. Ein gutes Planarisierungsvermögen der Polybenzoxazole ist ebenfalls von Vorteil. Durch die Verwendung eines gut planarisierenden Dielektrikums können nämlich bei der Herstellung von Bauteilen kostenintensive Schleifprozeduren (Chemical Mechanical Polishing; CMP) vermieden werden.

Aminophenole, die zur Herstellung von gut löslichen PBO-Vorstufen geeignet sind, sind beispielsweise aus der US-PS 4 525 539 und der EP 0 317 942 A2 bekannt. Hierbei gibt es jedoch keine Hinweise auf die Feuchteaufnahme oder das Planarisierungsverhalten der hergestellten Polymere nach der Cyclisierung auf dem Substrat (siehe EP 0 264 678 B1 und EP 0 317 942 A2). Bei der Herstellung der Aminophenole wird eine phenolische Ausgangsverbindung nitriert. Wenn dabei die Nitrierung nicht vollständig, d.h. zu 100 %, und vollkommen isomerenfrei erfolgt, d.h. nur in der o-Stellung zur Hydroxygruppe darf eine Nitrierung erfolgen, dann entstehen nach der Reduktion der Nitrogruppe teilweise Aminophenole, die in der PBO-Vorstufe keine vollständige Cyclisierung mehr zulassen und die Eigenschaften des Polybenzoxazols erheblich beeinträchtigen. Dies ist ein großer Nachteil der bekannten Herstellungsverfahren.

Aus der SU 1 205 518 A sind aromatische Aminophenole bekannt. Die aus diesen Aminophenolen hergestellten Polybenzoxazole zeigen aber keine hohe Temperaturstabilität (1 % Masseverlust bereits bei 420 bis 430°C). Außerdem wird bei der Herstellung der Aminophenole cancerogenes Hydrazinhydrat verwendet, was einen erheblichen Nachteil darstellt. Auch hier gibt es keine Hinweise auf die Feuchteaufnahme und das Planarisierungsverhalten der hergestellten Polymeren nach der Cyclisierung auf dem Substrat.

Ein Verfahren zur Herstellung von Bisaminophenolen ist auch aus "Polymer Preprints" 34 (1), 1993, Seiten 425 und 426 bekannt. Dieses Verfahren hat den Nachteil, daß es hohe Temperaturen erfordert, d.h. deutlich höhere Temperaturen als 100°C (Lösungen in Dimethylacetamid und Toluol werden zum Rückfluß erhitzt). Hohe Reaktionstemperaturen fördern jedoch Nebenreaktionen, welche die Ausbeute verringern (sie liegt bei maximal 73 %) und die Reinigung des erwünschten Produktes erschweren. Hierbei gibt es ebenfalls keine Hinweise auf die Feuchteaufnahme oder das Planarisierungsverhalten der hergestellten Polymeren nach der Cyclisierung auf dem Substrat.

Aufgabe der Erfindung ist es, Bis-o-aminophenole und Bis-o-aminothiophenole bereitzustellen, die zur Herstellung von Polymeren geeignet sind, welche die stark gestiegenen Anforderungen der Mikroelektronik erfüllen. Die Bis-o-amino(thio)-phenole sollen insbesondere die Herstellung gut löslicher Polymer-Vorstufen ermöglichen, die nach der Cyclisierung auf einem Substrat Polybenzoxazole bzw. Polybenzothiazole mit geringer Feuchteaufnahme, hoher Temperaturstabilität und hohem Planarisierungsgrad ergeben.

Dies wird erfindungsgemäß durch Bis-o-aminophenole und Bis-o-aminothiophenole folgender Struktur erreicht: 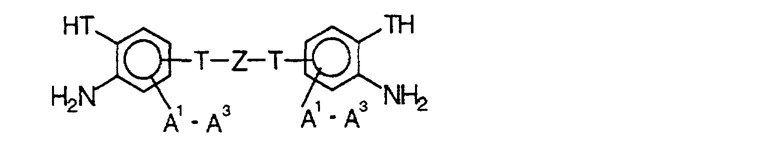 dabei gilt folgendes:
A¹ bis A³ sind - unabhängig voneinander - H, CH₃, OCH₃, CH₂CH₃ oder OCH₂CH₃;
T ist O oder S;
Z ist einer der folgenden Reste: 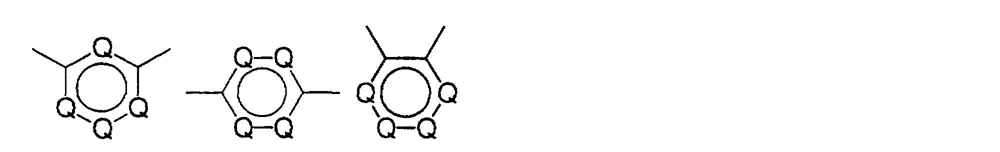 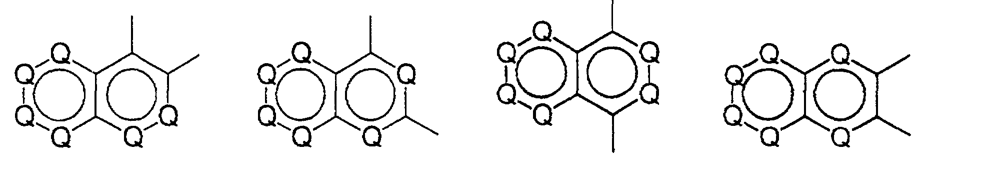 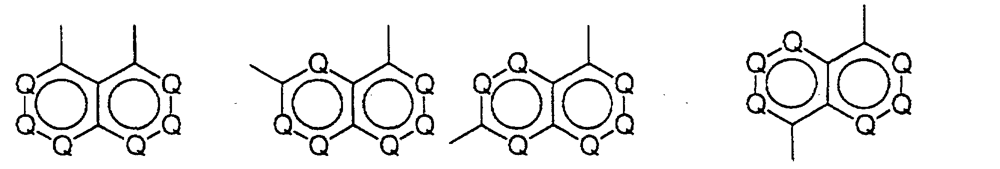 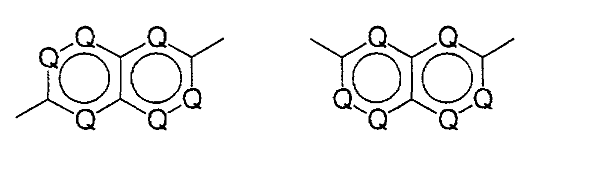 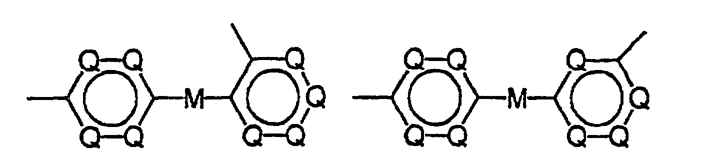 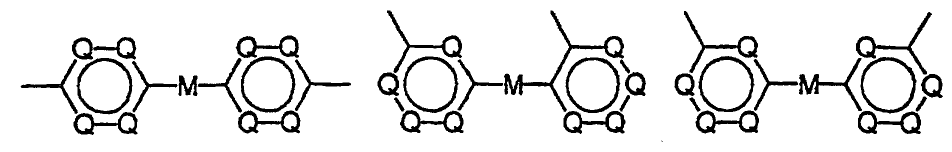 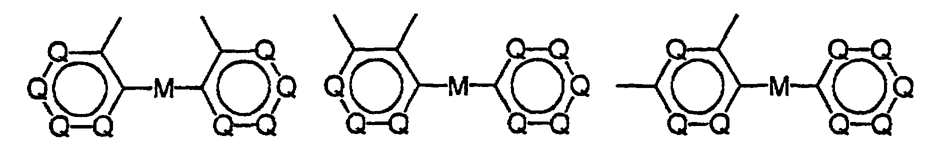 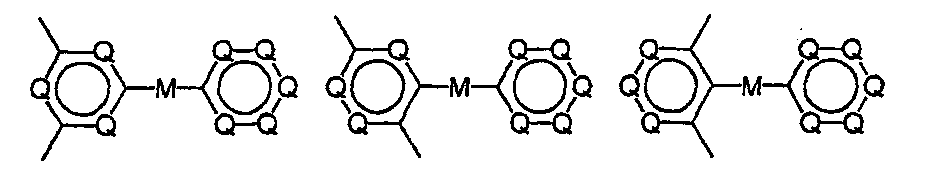 oder 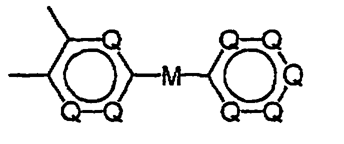 mit
   - Q =: C-A oder N,
   mit A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ mit p = bis 8 (Kette linear oder verzweigt), OC(CH₃)₃, OC(CF₃)₃, C₆H₅, C₆F₅, OC₆H₅, OC₆F₅, Cyclopentyl, Perfluorcyclopentyl, Cyclohexyl oder Perfluorcyclohexyl,
   wobei in den isolierten aromatischen Ringen maximal 3 N-Atome pro Ring vorhanden sein dürfen und nur 2 N-Atome benachbart sein können und in den anellierten Ringsystemen maximal 2 N-Atome pro Ring vorhanden sein dürfen,
   - M =: eine Einfachbindung, CH(CH₃), CH(CF₃), CF(CH₃), CF(CF₃), CH(C₆H₅), CH(C₆F₅), CF(C₆H₅), CF(C₆F₅), C(CH₃) (C₆H₅), C(CH₃) (C₆F₅), C(CF₃) (C₆H₅), C(CF₃)(C₆F₅), C(C₆R₅)₂, C(C₆F₅)₂, CO, SO₂, 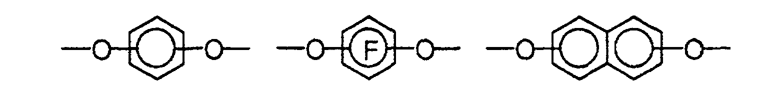 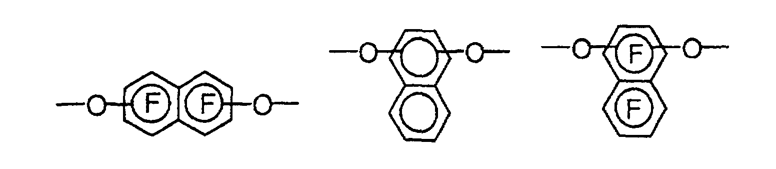 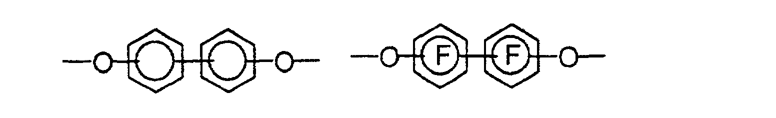 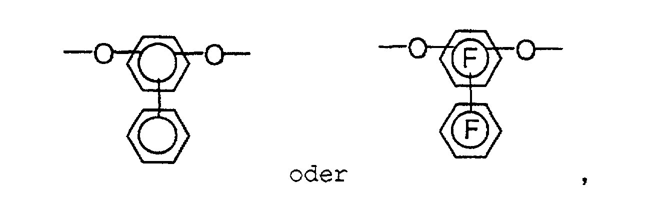 mit der Maßgabe, daß bei
   - Z =: 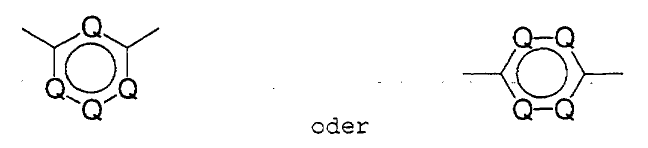 mindestens eine Gruppierung Q des Bis-o-aminophenols die Bedeutung N oder C-A mit A ≠ H haben muß und bei
   - Z =: 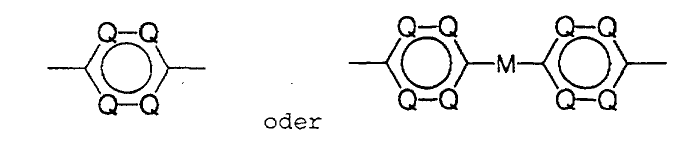 wenn Q die Bedeutung C-F hat und M eine Einfachbindung ist, sich die Aminofunktionen des Bis-o-aminophenols in o- oder p-Stellung zur O-Brücke befinden müssen.

Die neuen Verbindungen weisen beispielsweise folgende Struktur auf:

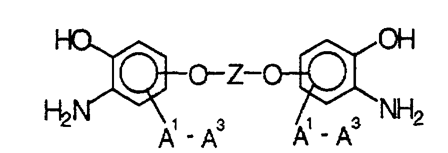

Bei derartigen Verbindungen sind offensichtlich die Etherbrücken für die gute Löslichkeit und die guten Planarisierungseigenschaften der damit hergestellen Polymer-Vorstufen verantwortlich. Im übrigen bedeutet die Charakterisierung "A¹-A³" in der Strukturformel, daß die Aminophenylgruppen Reste A¹, A² und A³ aufweisen.

Die Bis-o-amino(thio)phenole werden in der Weise hergestellt, daß
(a) eine Halogenverbindung der Struktur

   X-Z-X

   in einem Lösemittel bei einer Temperatur zwischen 20 und 100°C
   mit einem Nitrophenol bzw. Nitrothiophenol (kurz "Nitro-(thio)phenol") der Struktur 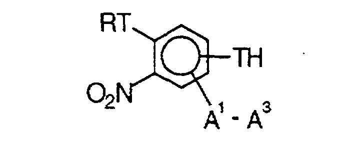
   in Gegenwart mindestens der stöchiometrischen Menge einer Base oder mit einem Alkalisalz des Nitro(thio)phenols zur Reaktion gebracht wird,
      wobei X ein Halogenatom ist, A¹ bis A³, T und Z die angegebene Bedeutung haben und R einer der folgenden Reste ist: Alkyl, Alkoxyalkyl, Alkenyl, Alkoxyalkenyl, Alkinyl oder Alkoxyalkinyl mit jeweils maximal 6 C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal 4 aliphatischen C-Atomen; und
   (b) die dabei gebildete Dinitroverbindung zur Diaminoverbindung reduziert und die Gruppe R abgespalten wird.

Die Verbindungen X-Z-X sind aromatische oder substituierte aromatische Verbindungen (mit X = Halogen). Dies können sowohl perhalogenierte als auch teilhalogenierte Verbindungen mit mindestens zwei Halogenatomen sein. Besonders bevorzugt werden teil- oder perfluorierte Verbindungen. Beispiele für derartige Verbindungen sind: 1,2,4-Trifluorbenzol, 2,3,5,6-Tetrafluortoluol, Octafluortoluol, 2,3,5,6-Tetrafluor-p-xylol, 2,6-Difluorpyridin, 2,3,5,6-Tetrafluorpyridin, Pentafluorpyridin, Decafluorbiphenyl und Decafluorbenzophenon.

Bei den Nitro(thio)phenolen ist wesentlich, daß sich der mit R geschützte Hydroxy- bzw. Mercaptorest in o-Stellung zur Nitrogruppe befindet. Die Herstellung von Nitro(thio)phenolen mit einer geschützten Hydroxy- oder Mercaptogruppe in o-Stellung zur Nitrogruppe ist in der gleichzeitig eingereichten deutschen Patentanmeldung Akt.Z. 197 42 135.0 - "o-Nitro-(thio) phenolderivate und deren Herstellung" (GR 97 P 3683) beschrieben.

Die Schutzgruppe R ist vorzugsweise eine Alkyl-, Alkoxyalkyl-, Phenyl- oder Benzylgruppe. Dabei ist wichtig, daß der Rest RT bei der Reaktion zwischen der Halogenverbindung und dem Nitro(thio)phenol stabil ist, anschließend aber gespalten werden kann.

Die Reaktion zwischen der Halogenverbindung und dem Nitro-(thio)phenol, bei der Ether- bzw. Thioetherbrücken gebildet werden, erfolgt in Gegenwart einer Base. Diese Base ist vorzugsweise ein Carbonat oder Hydrogencarbonat eines Alkali- oder Erdalkalimetalls, wie Natriumcarbonat und Kaliumcarbonat, oder eine organische Base mit einem tertiären N-Atom, beispielsweise Triethylamin und Pyridin.

Anstelle des Nitro(thio)phenols kann auch eines seiner Alkalisalze eingesetzt werden, beispielsweise das Kaliumsalz. In diesem Fall ist für die Umsetzung mit der Halogenverbindung keine Base erforderlich.

Als Reaktionstemperatur hat sich der Bereich von 20 bis 80°C als besonders geeignet erwiesen. Temperaturen ≤ 80°C werden wegen der höheren Selektivität der Umsetzung bevorzugt. Hierbei liegen die Ausbeuten nämlich über 90 %, was einen deutlichen Vorteil im Vergleich zum Stand der Technik darstellt.

Geeignete Lösemittel sind insbesondere Dimethylformamid, Diethylformamid, Dimethylacetamid, Dimethylsulfoxid, N-Methylpyrrolidon, γ-Butyrolacton, Acetonitril, Tetrahydrofuran und Pyridin. Prinzipiell können aber alle polaren aprotischen Lösemittel verwendet werden, in denen die Ausgangsverbindungen löslich sind.

Die Reduktion der Dinitroverbindung kann beispielsweise durch Hydrierung mit Wasserstoff an Pd/C durchgeführt werden. Es sind aber prinzipiell alle Verfahren geeignet, die sich für die Reduktion der Nitrogruppe zur Aminogruppe eignen.

Die Reduktion der Dinitroverbindung und die Abspaltung der Schutzgruppe R kann in zwei getrennten Verfahrensschritten erfolgen, wobei die Abspaltung der Schutzgruppe beispielsweise mit Trifluoressigsäure oder Titantetrachlorid durchgeführt wird. Vorzugsweise erfolgt jedoch die Reduktion der Nitrogruppe und die Abspaltung der Schutzgrupppe gleichzeitig, und zwar durch Hydrierung mit Wasserstoff an Pd/C. Die Hydrierung erfolgt vorzugsweise bei Temperaturen von 25 bis 50°C. Geeignete Lösemittel sind Ester oder Ether, beispielsweise Essigsäureethylester und Tetrahydrofuran.

Beim Verfahren nach der Erfindung, bei dem Nitro(thio)phenole eingesetzt werden, ergeben sich keine der Probleme, die beim Stand der Technik auftreten. So zeigen Polybenzoxazole, die sich beispielsweise von entsprechend diesem Verfahren hergestellten Bis-o-aminophenolen der Struktur 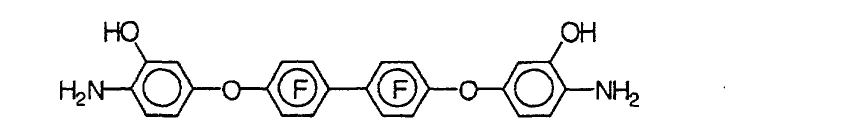 ableiten, trotz der strukturellen Ähnlichkeit deutlich höhere Temperaturstabilitäten (1 % Masseverlust erst bei 480°C) als die Polybenzoxazole entsprechend SU 1 205 518 A (1 % Masseverlust bereits bei 420 bis 430°C), bei denen sich die Hydroxygruppe in p-Stellung zur Etherbrücke befindet und die Aminogruppe in m-Stellung.

Die aus den Bis-o-amino(thio)phenolen nach der Erfindung hergestellten Polymer-Vorstufen sind in vielen organischen Lösemitteln, wie Aceton, Ethyllactat, N-Methylpyrrolidon, Diethylenglykolmono- bzw. -diethylether, Cyclohexanon und γ-Butyrolacton, sowie in metallionenfreien wäßrig-alkalischen Entwicklern gut löslich. Sie sind deshalb als Basispolymer für positiv photostrukturierbare und wäßrig-alkalisch entwickelbare Dielektrika gut geeignet. Die Vorstufen können mittels Schleudertechnik problemlos auf Substrate, wie Siliziumwafer, aufgebracht werden, sie bilden gleichmäßige Filme und lassen sich auf dem Substrat gut cyclisieren. Ein besonderer Vorteil der aus diesen Bis-o-amino(thio)phenolen hergestellten Vorstufen ist das hohe Planarisierungsvermögen und die geringe Feuchteaufnahme.

Anhand von Ausführungsbeispielen soll die Erfindung noch näher erläutert werden.

### Beispiel 1

### Herstellung von 4,4'-Bis(4-nitro-3-benzyloxy-phenoxy)-octafluorbiphenyl

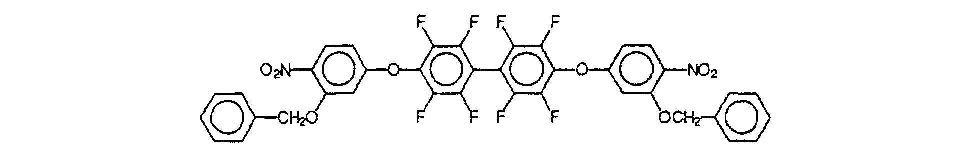

24,5 g 5-Hydroxy-2-nitro-phenylbenzylether (0,1 mol) und 16,7 g Decafluorbiphenyl (0,05 mol) werden in einem Dreihalskolben mit Rückflußkühler, Rührer und Stickstoffzuleitung in 270 ml trockenem Dimethylsulfoxid gelöst. Nach der Zugabe von 30 g Kaliumcarbonat (0,22 mol) wird die Lösung in einem regelbaren Ölbad 4 h auf 100°C erhitzt. Danach läßt man die Reaktionslösung auf Raumtemperatur abkühlen und filtriert den Rückstand über ein Faltenfilter ab. Die Lösung wird dann zu 500 ml Wasser gegeben und mit konzentrierter Salzäure bis zur sauren Reaktion versetzt. Das dabei ausfallende gelbbeige Reaktionsprodukt wird über einen Büchnertrichter abfiltriert, dreimal mit Wasser gewaschen und danach in einem Gemisch von Methanol und Methylenchlorid (Volumenverhältnis 1:1) umkristallisiert. Anschließend wird das Reaktionsprodukt in einem Vakuumschrank 48 h unter Stickstoff bei 40°C/10 mbar getrocknet (Ausbeute: 94 %).

Charakterisierung:
- Massenspektrum: Molekülpeak bei 784
- Elementaranalyse:

| | | | |
|---|---|---|---|
| Theoretischer Wert (in %) | C: 58,2 | H: 2,6 | N: 3,6 |
| Ermittelter Wert (in %) | C: 58,2 | H: 2,5 | N: 3,7 |

- Schmp: 212°C.

### Beispiel 2

### Herstellung von 4,4-Bis(4-amino-3-hydroxy-phenoxy)-octafluorbiphenyl

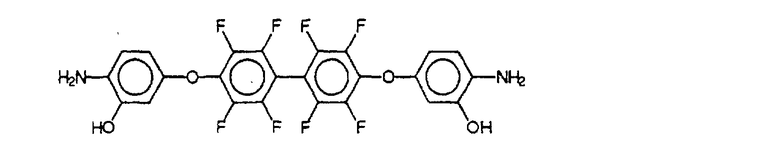

72 g des entsprechend Beispiel 1 hergestellten 4,4'-Bis-(4-nitro-3-benzyloxy-phenoxy)-octafluorbiphenyl (0,09 mol) werden in 600 ml eines Gemisches von Tetrahydrofuran und Essigsäureethylester (Volumenverhältnis 1:1) gelöst, und zu der Lösung werden 7 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 7 Tagen wird die Reaktion beendet. Die gelbbeige Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 96 %).

Charakterisierung:
- Massenspektrum: Molekülpeak bei 544
- Elementaranalyse:

| | | | |
|---|---|---|---|
| Theoretischer Wert (in %) | C: 53,0 | H: 2,2 | N: 5,1 |
| Ermittelter Wert (in %) | C: 52,8 | H: 2,3 | N: 5,2 |

- Schmp: 245°C.

### Beispiel 3

### Herstellung von 4,4'-Bis(4-nitro-3-benzyloxy-phenoxy)-octafluorbenzophenon

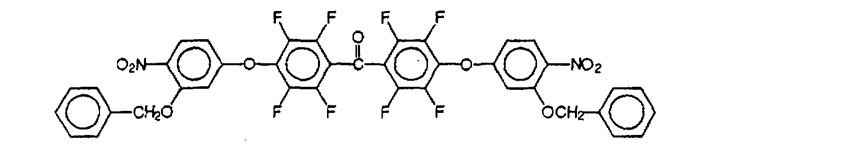

28,3 g des Kaliumsalzes von 5-Hydroxy-2-nitro-phenylbenzylether (0,1 mol) und 18,1 g Decafluorbenzophenon (0,05 mol) werden in einem Dreihalskolben mit Rückflußkühler, Rührer und Stickstoffzuleitung in 300 ml trockenem Acetonitril gelöst. Die Lösung wird dann 24 h bei Raumtemperatur gerührt und danach über ein Faltenfilter abfiltriert. Die klare Lösung wird anschließend in einem Rotationsverdampfer auf die Hälfte eingeengt, wobei ein gelbbeiges Reaktionsprodukt ausfällt. Anschließend wird das Reaktionsprodukt in Essigsäureethylester umkristallisiert und dann in einem Vakuumschrank 48 h unter Stickstoff bei 40°C/10 mbar getrocknet (Ausbeute: 92 %).

Charakterisierung:
- Massenspektrum: Molekülpeak bei 812
- Elementaranalyse:

| | | | |
|---|---|---|---|
| Theoretischer Wert (in %) | C: 57,6 | H: 2,5 | N: 3,4 |
| Ermittelter Wert (in %) | C: 57,4 | H: 2,6 | N: 3,4 |

- Schmp: 167°C.

### Beispiel 4

### Herstellung von 4,4'-Bis(4-amino-3-hydroxy-phenoxy)-octafluorbenzophenon

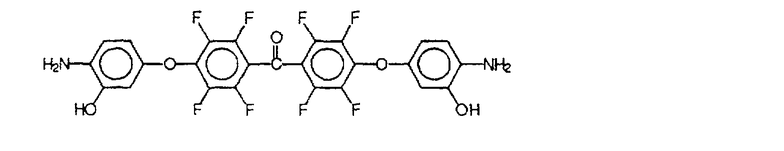

32,5 g des entsprechend Beispiel 3 hergestellten 4,4'-Bis-(4-nitro-3-benzyloxy-phenoxy)-octafluorbenzophenon (0,04 mol) werden in 500 ml eines Gemisches von Tetrahydrofuran und Essigsäureethylester (Volumenverhältnis 1:1) gelöst, und zu der Lösung werden 3 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die gelbbeige Lösung wird in einem Rotationsverdampfer auf die Hälfte eingeengt, dann werden 100 ml n-Hexan zugegeben; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 95 %).

Charakterisierung:
- Massenspektrum: Molekülpeak bei 572
- Elementaranalyse:

| | | | |
|---|---|---|---|
| Theoretischer Wert (in %) | C: 52,5 | H: 2,1 | N: 4,9 |
| Ermittelter Wert (in %) | C: 52,6 | H: 2,0 | N: 5,0 |

- Schmp: 208°C (Zersetzung).

### Beispiel 5

### Herstellung von 2,4-Bis(4-nitro-3-benzyloxy-phenoxy)-3,5,6-trifluorpyridin

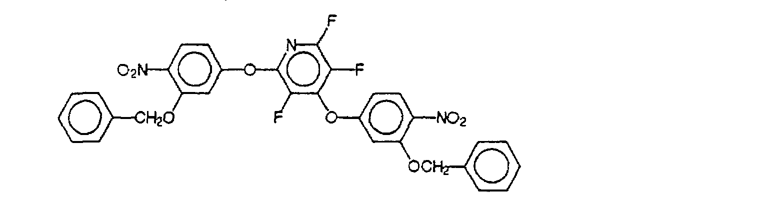

56,6 g des Kaliumsalzes von 5-Hydroxy-2-nitro-phenylbenzylether (0,2 mol) werden in 200 ml Dimethylformamid gelöst und unter Rühren langsam in eine Lösung von 16,9 g Pentafluorpyridin (0,1 mol) in 200 ml Dimethylformamid getropft. Dann wird 5 Tage bei Raumtemperatur gerührt und die Reaktionslösung anschließend über ein Faltenfilter abfiltriert. Die erhaltene klare Lösung wird zu 800 ml Wasser gegeben und mit konzentrierter Salzsäure bis zur sauren Reaktion versetzt. Aus der anfangs milchigen Lösung fällt ein weißes Reaktionsprodukt aus, das abfiltriert und dann zuerst dreimal mit Wasser und danach mit einem Gemisch von Diethylether und Methylenchlorid (Volumenverhältnis 1:1) gewaschen wird. Anschließend wird das Reaktionsprodukt in einem Vakuumschrank 48 h unter Stickstoff bei 40°C/10 mbar getrocknet (Ausbeute: 93 %).

Charakterisierung:
- Massenspektrum: Molekülpeak bei 619
- Elementaranalyse:

| | | | |
|---|---|---|---|
| Theoretischer Wert (in %) | C: 60,1 | H: 3,3 | N: 6,8 |
| Ermittelter Wert (in %) | C: 59,9 | H: 3,4 | N: 6,9 |

- Schmp.: 135°C.

### Beispiel 6

### Herstellung von 2,4-Bis(4-amino-3-hydroxy-phenoxy)-3,5,6-trifluorpyridin

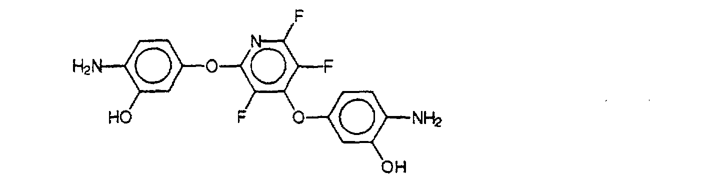

99 g des entsprechend Beispiel 5 hergestellten 2,4-Bis-(4-nitro-3-benzyloxy-phenoxy)-3,5,6-trifluorpyridin (0,16 mol) werden in 600 ml eines Gemisches von Tetrahydrofuran und Essigsäureethylester (Volumenverhältnis 1:1) gelöst, und zu der Lösung werden 10 g Pd/C (Palladium/Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die gelbbeige Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 93 %).

Charakterisierung:
- Massenspektrum: Molekülpeak bei 379
- Elementaranalyse:

| | | | |
|---|---|---|---|
| Theoretischer Wert (in %) | C: 53,8 | H: 3,2 | N: 11,1 |
| Ermittelter Wert (in %) | C: 53,7 | H: 3,2 | N: 11,1 |

### Beispiel 7

### Herstellung von 2,4-Bis(4-nitro-3-benzyloxy-phenoxy)-1-trifluormethyl-3,5,6-trifluorbenzol

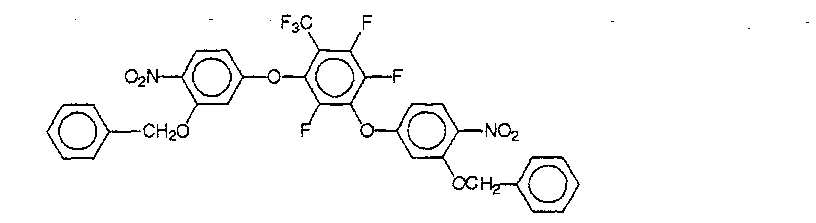

28,3 g des Kaliumsalzes von 5-Hydroxy-2-nitro-phenylbenzylether (0,1 mol) werden in 200 ml Dimethylacetamid gelöst und bei 0°C unter Rühren langsam in eine Lösung von 23,6 g Octafluortoluol (0,1 mol) in 200 ml Dimethylacetamid getropft. Dann wird 1 h bei Raumtemperatur und 3 Tage bei 50°C gerührt und die Reaktionslösung anschließend über ein Faltenfilter abfiltriert. Die erhaltene klare Lösung wird zu 600 ml Wasser gegeben und mit konzentrierter Salzsäure bis zur sauren Reaktion versetzt. Aus der anfangs milchigen Lösung fällt ein weißes Reaktionsprodukt aus, das abfiltriert und dann zuerst dreimal mit Wasser und danach mit einem Gemisch von Diethylether und Methylenchlorid (Volumenverhältnis 1:1) gewaschen wird. Anschließend wird das Reaktionsprodukt in einem Vakuumschrank 48 h unter Stickstoff bei 40°C/10 mbar getrocknet (Ausbeute: 92 %).

Charakterisierung:
- Massenspektrum: Molekülpeak bei 686
- Elementaranalyse:

| | | | |
|---|---|---|---|
| Theoretischer Wert (in %) | C: 57,7 | H: 2,9 | N: 4,1 |
| Ermittelter Wert (in %) | C: 57,8 | H: 2,8 | N: 4,2 |

### Beispiel 8

### Herstellung von 2,4-Bis(4-amino-3-hydroxy-phenoxy)-1-trifluormethyl-3,5,6-trifluorbenzol

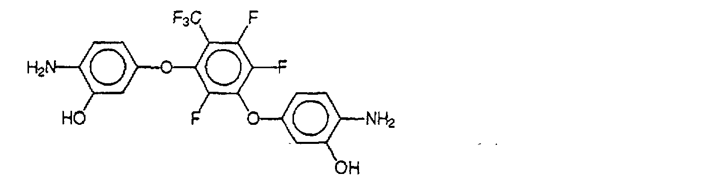

103 g des entsprechend Beispiel 7 hergestellten 2,4-Bis(4-nitro-3-benzyloxy-phenoxy)-1-trifluormethyl-3,5,6-tri-fluorbenzol (0,15 mol) werden in 600 ml eines Gemisches von Tetrahydrofuran und Essigsäureethylester (Volumenverhältnis 1:1) gelöst, und zu der Lösung werden 10 g Pd/C (Palladium/ Kohlenstoff) gegeben. Dann wird bei Raumtemperatur in einem Autoklaven unter starkem Rühren mit Wasserstoff bei einem Druck von 1 bar hydriert; nach 3 Tagen wird die Reaktion beendet. Die gelborange Lösung wird in einem Rotationsverdampfer auf die Hälfte eingeengt und über Nacht bei Raumtemperatur stehengelassen; dabei fällt das Reaktionsprodukt kristallin aus. Anschließend wird das Reaktionsprodukt abgetrennt und in einem Vakuumschrank 48 h unter Stickstoff bei 40°C/10 mbar getrocknet (Ausbeute: 95 %).

Charakterisierung:
- Massenspektrum: Molekülpeak bei 446
- Elementaranalyse:

| | | | |
|---|---|---|---|
| Theoretischer Wert (in %) | C: 51,1 | H: 2,7 | N: 6,3 |
| Ermittelter Wert (in %) | C: 50,8 | H: 2,8 | N: 6,3 |

## Patentansprüche

1. Bis-o-aminophenole und Bis-o-aminothiophenole der Struktur 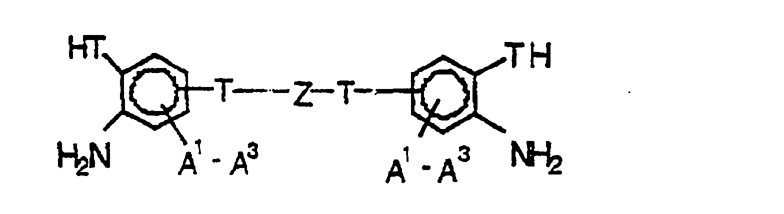 wobei folgendes gilt:
A¹ bis A³ sind - unabhängig voneinander - H, CH₃, OCH₃, CH₂CH₃, oder OCH₂CH₃;
T ist O oder S;
Z ist einer der folgenden Reste: 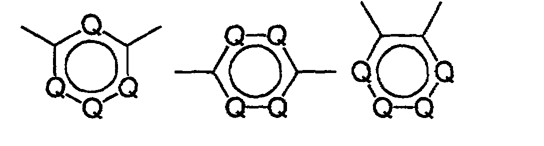 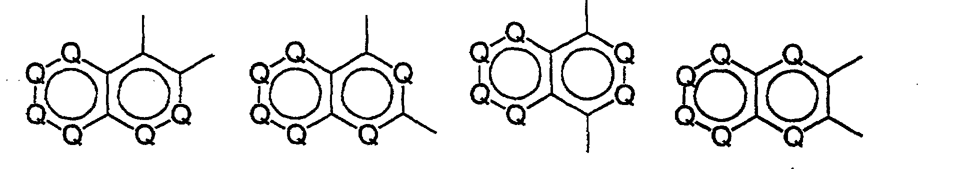 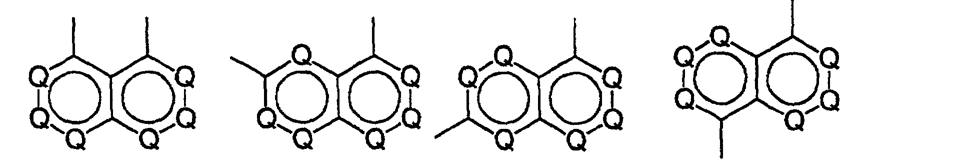 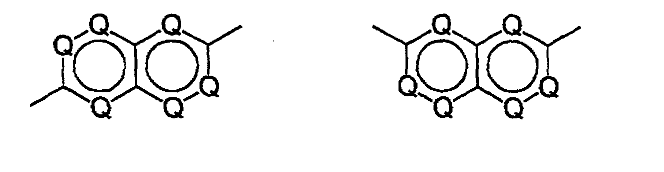 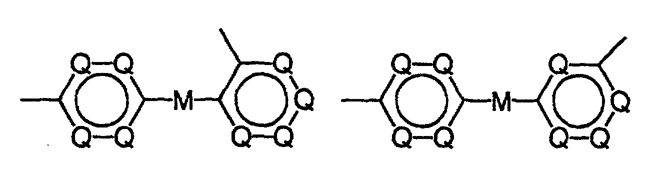 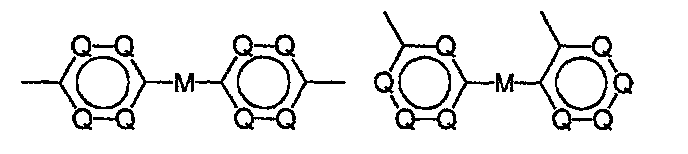 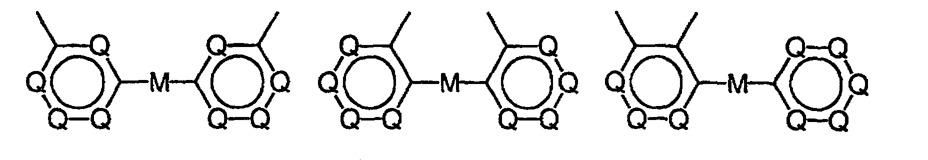 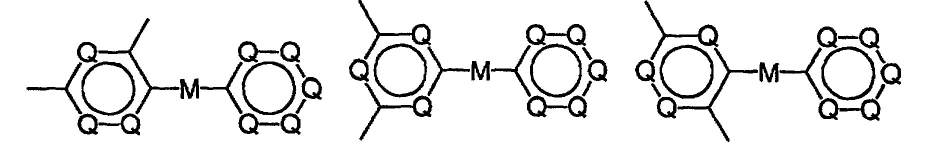 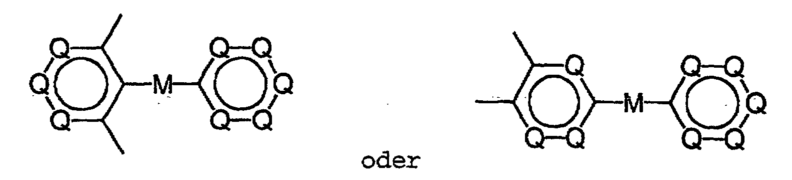 mit
Q = C-A oder N,
mit A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ mit p = 0 bis 8 (Kette linear oder verzweigt), OC(CH₃)₃, OC(CF₃)₃, C₆H₅, C₆F₅, OC₆H₅, OC₆F₅, Cyclopentyl, Perfluorcyclopentyl, Cyclohexyl oder Perfluorcyclohexyl,
wobei in den isolierten aromatischen Ringen maximal 3 N-Atome pro Ring vorhanden sein dürfen und nur 2 N-Atome benachbart sein können und in den anellierten Ringsystemen maximal 2 N-Atome pro Ring vorhanden sein dürfen,
M = eine Einfachbindung, CH(CH₃), CH(CF₃), CF(CH₃), CF(CF₃), CH(C₆H₅), CH(C₆F₅), CF(C₆H₅), CF(C₆F₅), C(CH₃) (C₆H₅), C(CH₃) (C₆F₅), C(CF₃) (C₆H₅), C(CF₃) (C₆F₅), C(C₆H₅)₂, C(C₆F₅)₂, CO, SO₂, 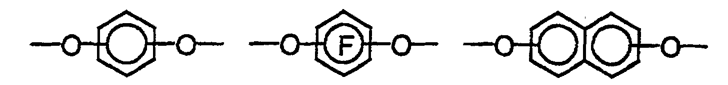 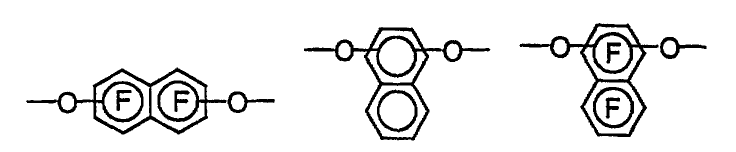 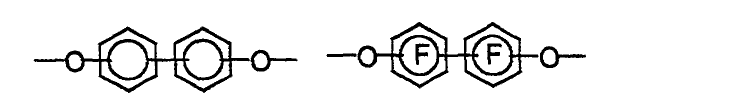 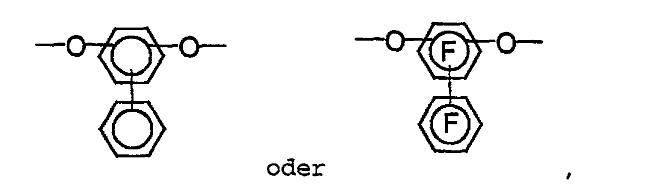
mit der Maßgabe, daß bei
Z = 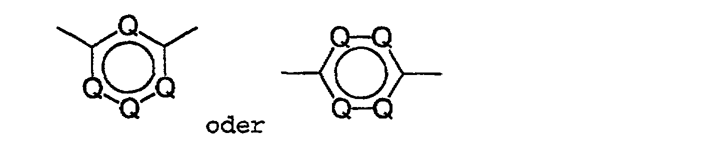 mindestens eine Gruppierung Q des Bis-o-aminophenols die Bedeutung N oder C-A mit A ≠ H haben muß und bei
Z = 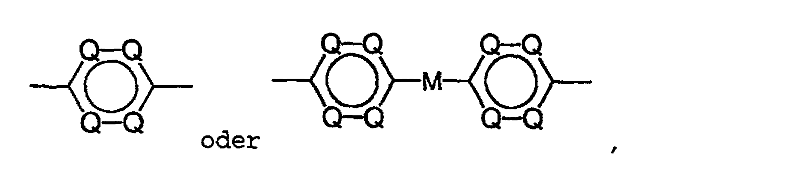 wenn Q die Bedeutung C-F hat und M eine Einfachbindung ist, sich die Aminofunktionen des Bis-o-aminophenols in o- oder p-Stellung zur O-Brücke befinden müssen.

2. Verfahren zur Herstellung von Bis-o-aminophenolen und Bis-o-aminothiophenolen nach Anspruch 1, **dadurch gekennzeichnet, daß**
(a) eine Halogenverbindung der Struktur
X-Z-X
in einem Lösemittel bei einer Temperatur zwischen 20 und 100°C
mit einem Nitrophenol bzw. Nitrothiophenol der Struktur 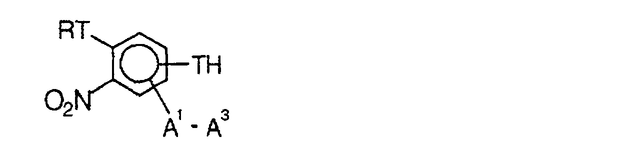 in Gegenwart mindestens der stöchiometrischen Menge einer Base oder mit einem Alkalisalz des Nitro(thio)phenols zur Reaktion gebracht wird,
wobei X ein Halogenatom ist, A¹ bis A³, T und Z die angegebene Bedeutung haben und R einer der folgenden Reste ist: Alkyl, Alkoxyalkyl, Alkenyl, Alkoxyalkenyl, Alkinyl oder Alkoxyalkinyl mit jeweils maximal 6 C-Atomen, Phenyl, Phenacyl, Benzyl und Benzylalkyl, Benzylalkenyl, Benzyloxyalkyl, Benzyloxyalkenyl, Benzylalkoxyalkyl oder Benzylalkoxyalkenyl mit jeweils maximal 4 aliphatischen C-Atomen; und
(b) die dabei gebildete Dinitroverbindung zur Diaminoverbindung reduziert und die Gruppe R abgespalten wird.

3. Verfahren nach Anspruch 2, **dadurch gekennzeichnet, daß** als Base ein Carbonat oder Hydrogencarbonat eines Alkali- oder Erdalkalimetalls eingesetzt wird.

4. Verfahren nach Anspruch 2, **dadurch gekennzeichnet, daß** eine organische Base mit einem tertiären N-Atom eingesetzt wird.

5. Verfahren nach einem der Ansprüche 2 bis 4, **dadurch gekennzeichnet, daß** die Reduktion und die Abspaltung der Gruppe R durch Wasserstoff erfolgt und mit Pd/C katalysiert wird.

## Claims

1. bis-o-Aminophenols or bis-o-aminothiophenols of the structure 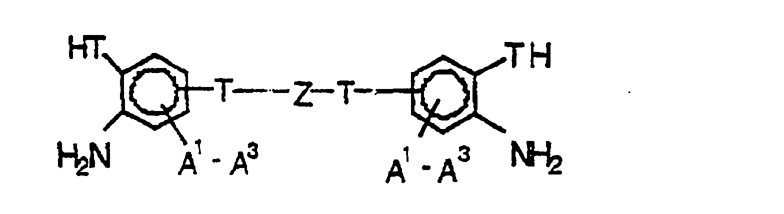 where:
A¹ to A³ are - independently of one another - H, CH₃, OCH₃, CH₂CH₃ or OCH₂CH₃ ;
T is O or S;
Z is one of the following radicals: 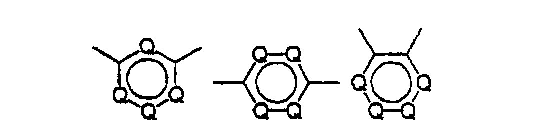 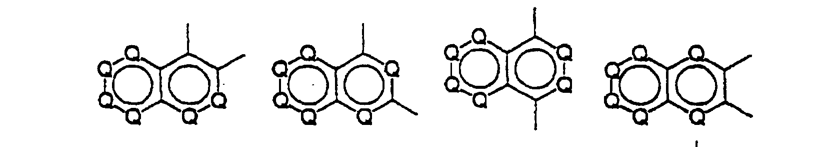 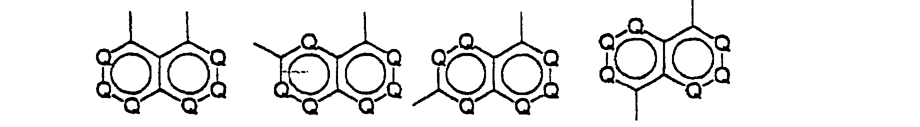 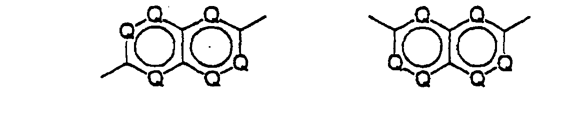 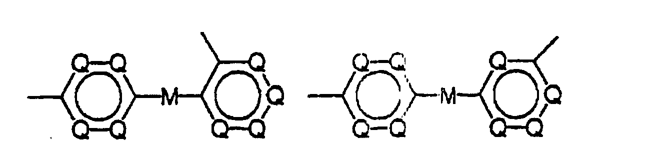 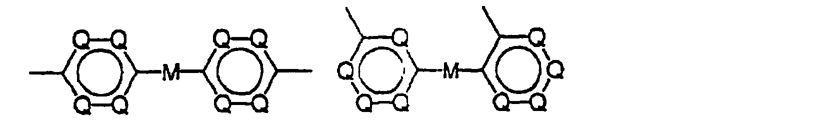 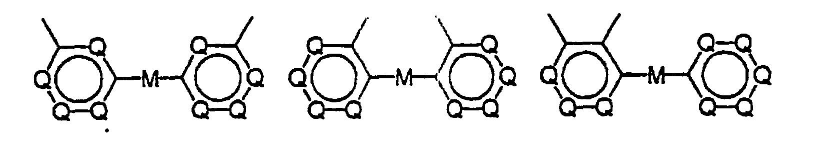 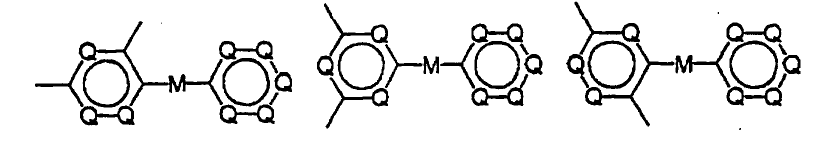 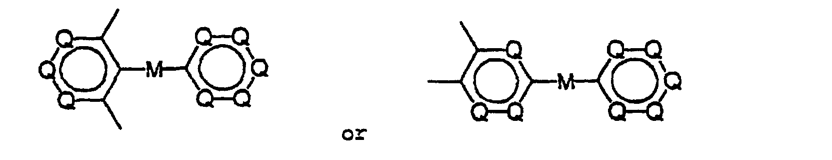
where Q = C-A or N,
where A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ where p = 0 to 8 (linear or branched chain), OC(CH₃)₃, OC(CF₃)₃, C₆H₅, C₆F₅, OC₆H₅, OC₆F₅, cyclopentyl, perfluorocyclopentyl, cyclohexyl or perfluorocyclohexyl,
where, in the isolated aromatic rings, a maximum of 3 N atoms may be present per ring and only 2 N atoms may be adjacent, and, in the fused ring systems, a maximum of 2 N atoms may be present per ring,
M = a single bond, CH(CH₃), CH(CF₃), CF(CH₃), CF(CF₃), CH(C₆H₅), CH(C₆F₅), CF(C₆H₅), CF(C₆F₅), C(CH₃)(C₆H₅), C(CH₃)(C₆F₅), C(CF₃)(C₆H₅), C(CF₃)(C₆F₅), C(C₆H₅)₂, C(C₆F₅)₂, CO, SO₂, 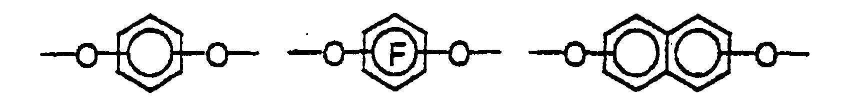 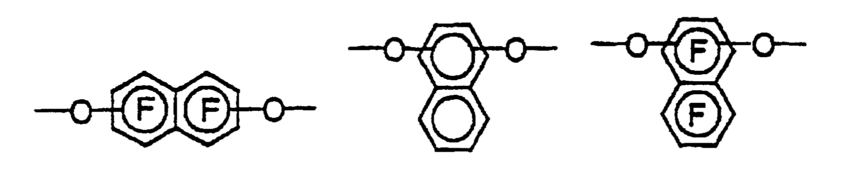 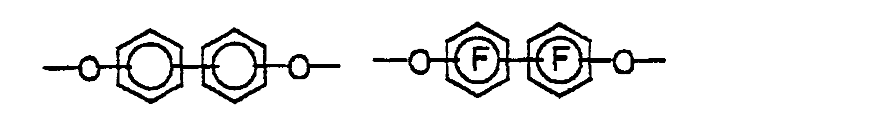 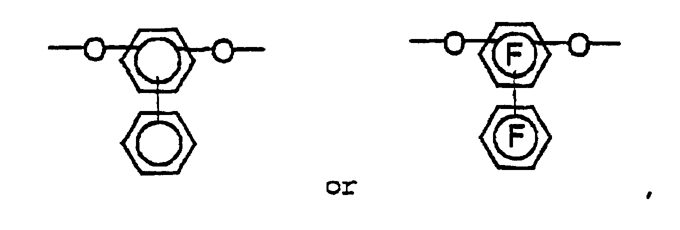
with the proviso that, when
Z = 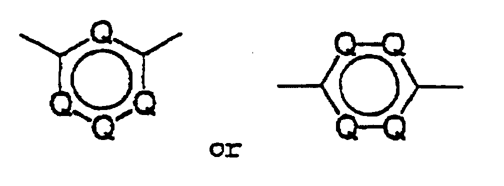 at least one group Q of the bis-o-aminophenol must be N or C-A, where A ≠ H, and when
Z = 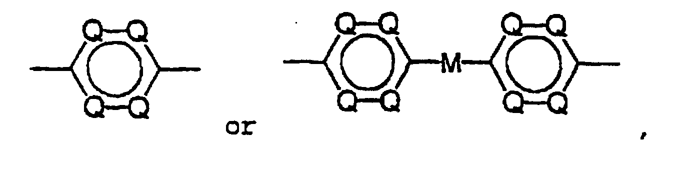 where Q is C-F and M is a single bond, the amino functions of the bis-o-aminophenol must be in the o- or p-position to the O bridge.

2. Process for the preparation of bis-o-aminophenols or bis-o-aminothiophenols according to Claim 1, **characterized in that**
(a) a halogen compound of the structure
X-Z-X
is reacted with a nitrophenol or nitrothiophenol of the structure 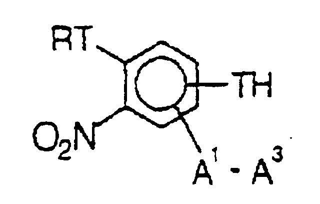 in the presence of at least a stoichiometric amount of a base, or with an alkali metal salt of the nitro(thio)phenol, in a solvent at a temperature between 20 and 100°C,
where X is a halogen atom, A¹ to A³, T and Z are as defined above, and R is one of the following radicals: alkyl, alkoxyalkyl, alkenyl, alkoxyalkenyl, alkynyl or alkoxyalkynyl, each having a maximum of 6 carbon atoms, phenyl, phenacyl, benzyl, benzylalkyl, benzylalkenyl, benzyloxyalkyl, benzyloxyalkenyl, benzylalkoxyalkyl or benzylalkoxyalkenyl, each having a maximum of 4 aliphatic carbon atoms; and
(b) the resultant dinitro compound is reduced to the diamino compound and the group R is removed.

3. Process according to Claim 2, **characterized in that** the base used is a carbonate or hydrogencarbonate of an alkali metal or alkaline earth metal.

4. Process according to Claim 2, **characterized in that** an organic base containing a tertiary N atom is used.

5. Process according to any one of Claims 2 to 4, **characterized in that** the reduction and the removal of the group R are carried out by means of hydrogen and are catalysed by Pd/C.

## Revendications

1. Bis-o-aminophénol et bis-o-aminothiophénol de formule : 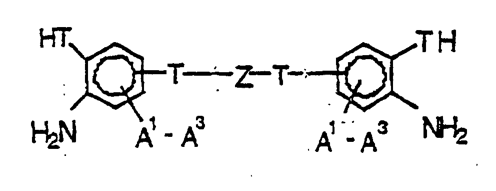 dans laquelle :
A¹ à A³ sont indépendamment les uns des autres H, CH₃, OCH₃, CH₂CH₃ ou OCH₂CH₃ ;
T est O ou S ;
Z est l'un des radicaux suivants: 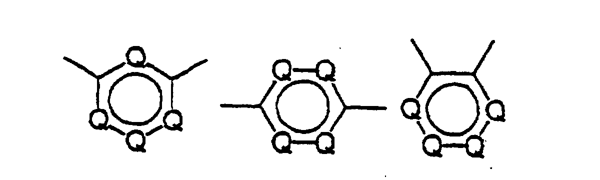 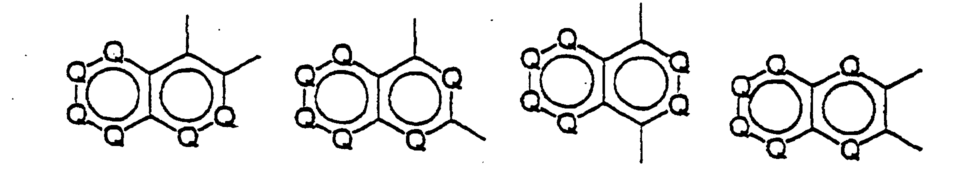 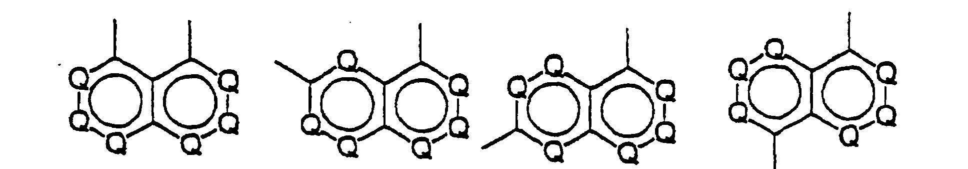 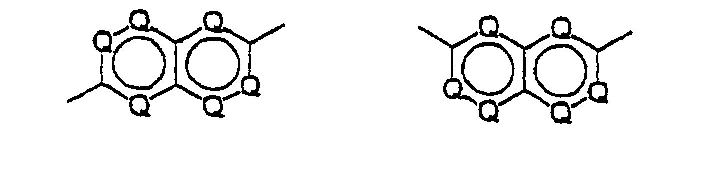 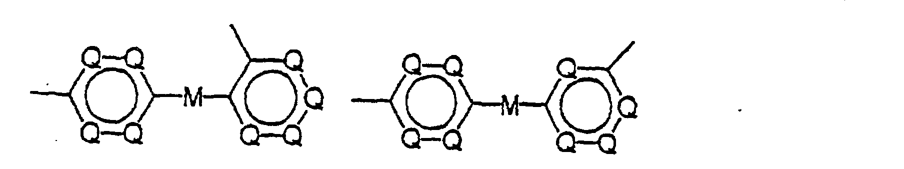 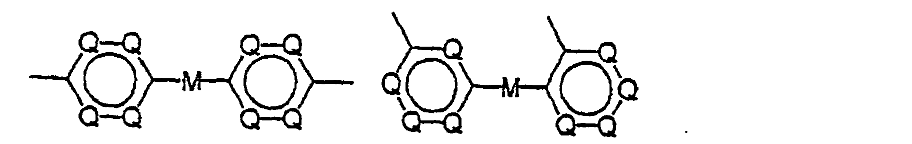 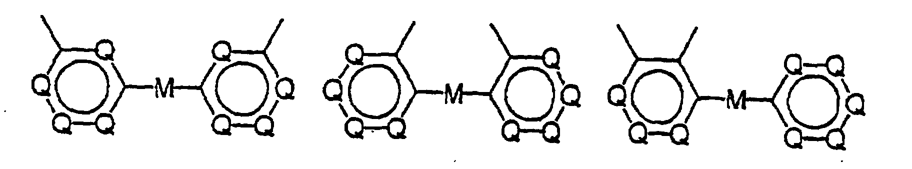 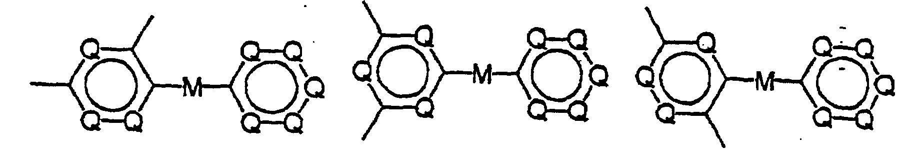 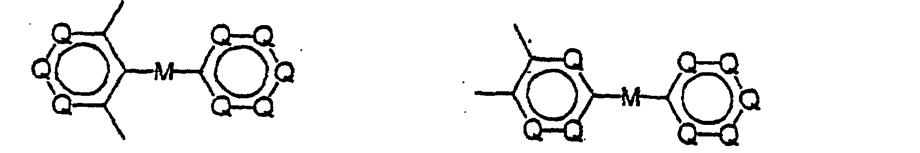
où Q = C-A ou N,
avec A = H, F, (CH₂)ₚCH₃, (CF₂)ₚCF₃, O(CH₂)ₚCH₃, O(CF₂)ₚCF₃, CO(CH₂)ₚCH₃, CO(CF₂)ₚCF₃ avec p = 0 à 8 (chaîne linéaire ou ramifiée), OC(CH₃)₃, OC(CF₃)₃, C₆H₅, C₆F₅, OC₆H₅, OC₆F₅, cyclopentyle, perfluorocyclopentyle, cyclohexyle ou perfluorocyclohexyle, dans lesquels il peut y avoir dans les cycles aromatiques isolés au maximum 3 atomes d'azote par cycle et seulement 2 atomes d'azote peuvent être voisins et dans les systèmes cycliques condensés il peut y avoir au maximum 2 atomes d'azote par cycle,
M = une simple liaison, CH(CH₃), CH(CF₃), CF(CH₃), CF(CF₃), CH(C₆H₅), CH(C₆F₅), CF(C₆H₅), CF(C₆F₅), C(CH₃)(C₆H₅), C(CH₃)(C₆F₅), C(CF₃)(C₆H₅), C(CF₃(C₆F₅), C(C₆H₅)₂, C(C₆F₅)₂, CO, SO₂, 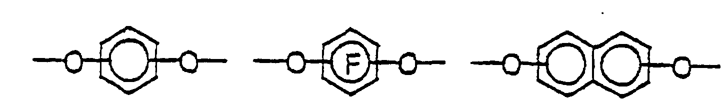 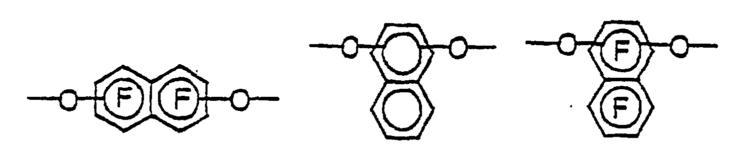 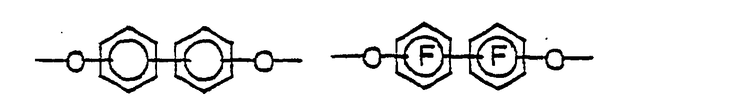 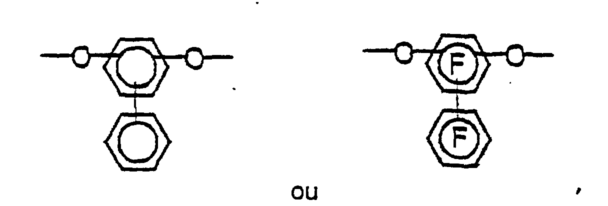
avec la condition que pour
Z= 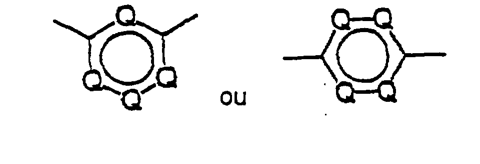 au moins un groupement Q du bis-o-aminophénol doit avoir la signification N ou C-A avec A différent de H et pour
Z= 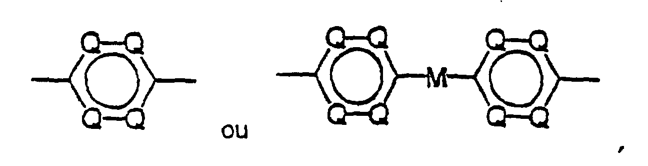 lorsque Q a la signification C-F et M est une simple liaison, les fonctions amino du bis-o-aminophénol doivent se trouver en position ortho ou en position para par rapport au pont O.

2. Procédé de préparation de bis-o-aminophénol et de bis-o-thiophénol suivant la revendication 1, **caractérisé en ce que** :
a) on met à réagir un composé halogéné de formule X-Z-X, dans un solvant à une température comprise entre 20 et 100°C sur un nitrophénol ou un nitrothiophénol de formule : 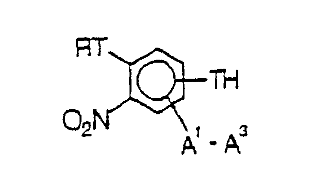 en présence d'au moins la quantité stoechiométrique d'une base ou sur un sel de métal alcalin du nitro(thio)phénol,
X étant un atome d'halogène, A¹ à A³, T et Z ayant la signification indiquée et R étant l'un des radicaux suivants : alkyle, alcoxyalcoyle, alcényle, alcoxyalcényle, alcinyle ou alcoxyalcinyle, ayant respectivement au maximum 6 atomes de carbone, phényle, phénacyle, benzyle et benzylalcoyle, benzylalcényle, benzyloxyalcoyle, benzyloxyalcényle, benzylalcoxy-alcoyle ou benzylalcoxyalcényle ayant respectivement au maximum 4 atomes de carbone aliphatique ; et
b) on réduit le composé dinitro ainsi formé en le composé diamino et on élimine le groupe R.

3. Procédé suivant la revendication 2, **caractérisé en ce qu'**on utilise comme base un carbonate ou un hydrogéno-carbonate d'un métal alcalin ou d'un métal alcalino-terreux.

4. Procédé suivant la revendication 2, **caractérisé en ce que** l'on utilise une base organique ayant un atome d'azote tertiaire.

5. Procédé suivant l'une des revendications 2 à 4, **caractérisé en ce que** l'on effectue la réduction et l'élimination du groupe R par dé l'hydrogène et par catalyse sur Pt/C.
